# EUROPEAN PATENT APPLICATION

(11) **EP 0 923 943 A1**
(43) Date of publication of application: **23.06.1999**
(21) Application number: 97920943.4
(22) Date of filing: 09.05.1997
(51) Int. Cl.: A61K 47/36

(54) **MICELLAR AQUEOUS COMPOSITION AND METHOD FOR SOLUBILIZING HYDROPHOBIC DRUG**

(30) Priority: 09.05.1996 JP 11511696
(71) Applicant: TAISHO PHARMACEUTICAL CO. LTD, Tokyo 170-8633 (JP)
(72) Inventor: YAMAHIRA, Tomohiro, Taisho Pharmaceutical Co.,Ltd., Tokyo 170-8633 (JP); MIWA, Akio, Taisho Pharmaceutical Co.,Ltd., Tokyo 170-8633 (JP); ISHIBE, Atsuo, Taisho Pharmaceutical Co.,Ltd., Tokyo 170-8633 (JP); ITAI, Shigeru, Taisho Pharmaceutical Co.,Ltd., Tokyo 170-8633 (JP)
(74) Representative: Kraus, Walter, Dr.
(86) International application number: JP9701566
(87) International publication number: WO9741894

(57) **Abstract**

The present invention relates to: micellar aqueous compositions formed by chitosan derivatives in which carboxy-C₁₋₅ alkyl groups have been introduced as the hydrophilic groups and alkyl groups of 9 to 21 carbon atoms or aliphatic acyl groups of 9 to 21 carbon atoms have been introduced as the hydrophobic groups, and hydrophobic drugs; and a method of solubilizing hydrophobic drugs by adding the chitosan derivatives and hydrophobic drugs into an aqueous solvent to form micellar aqueous compositions. According to the present invention, drugs that could not be used for injection due to their insolubility in water can be formulated into injections, and also the effect of promoting absorptivity of drugs that were poorly absorbed orally etc. due to its poor solubility can be expected.

## Description

### Technical Field

The present invention relates to a technology for solubilizing hydrophobic drugs using chitosan derivatives into which hydrophilic and hydrophobic groups have been introduced.

### Background Art

Solubilization of hydrophobic drugs using surfactants, cyclodextrins, or other polymer compounds have been conventionally reported.

For chitosan derivatives, Japanese Examined Patent Publication (Kokoku) No. 63-28414 discloses pharmaceutical preparations wherein the dissolution property of hydrophobic drugs has been improved using chitins and/or chitosans, Japanese Unexamined Patent Publication (Kokai) No. 2-131434 discloses pharmaceutical preparations wherein the solubility and the dissolution property of hydrophobic drugs in water have been improved using low molecular weight chitosans, and Japanese Unexamined Patent Publication (Kokai) No. 4-18036 discloses pharmaceutical preparations wherein the solubility and the dissolution property of hydrophobic drugs in water have been improved using alkali-soluble low molecular weight chitosans and alkali-insoluble high molecular weight chitosans.

However, even the use of the above-mentioned chitins or chitosans does not permit the formation of micellar aqueous compositions, and thereby the formulation of hydrophobic drugs as injections are difficult.

A recent report disclosed a technology in which hydrophobic drugs were solubilized using chitosan derivatives. But, there have been no examples in which hydrophobic drugs have been solubilized using chitosan derivatives in which carboxymethyl groups have been introduced.

### Disclosure of the Invention

It is an object of the present invention to provide a pharmaceutical composition containing a hydrophobic drug capable of forming a micellar aqueous composition. The composition enables the formulation of drugs which could not be used as an injection due to its poor solubility in water and the accelerated absorption of drugs which have poor absorptivity due to poor solubility as orally administered preparations.

After intensive research to attain the above objective, the inventors of the present invention have discovered that chitosan derivatives in which carboxy-C₁₋₅ alkyl groups have been introduced as the hydrophilic groups and alkyl groups of 9 to 21 carbon atoms or aliphatic acyl groups of 9 to 21 carbon atoms have been introduced as the hydrophobic groups form micellar aqueous compositions with hydrophobic drugs, and have accomplished the present invention.

Thus, the first invention of the present application is a pharmaceutical composition comprising
(1) a hydrophobic drug, and
(2) a chitosan derivative represented by formula (I): 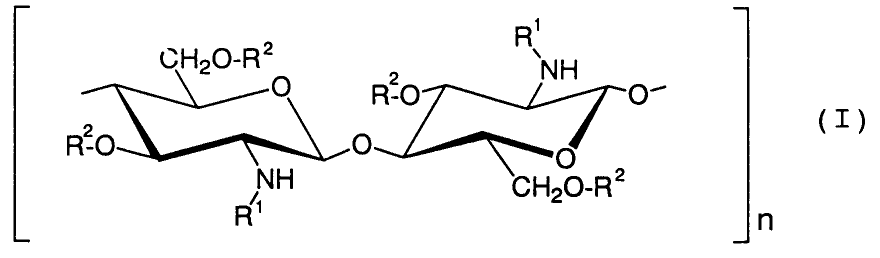
   wherein R¹ represents a hydrogen atom, an acetyl group, an alkyl group of 9 to 21 carbon atoms, or an aliphatic acyl group of 9 to 21 carbon atoms, and R² represents a hydrogen atom or a carboxy-C₁₋₅ alkyl group; and
   the degree of deacetylation of an N-acetyl group represented by R¹ is 70 to 100% with 100% being defined as one deacetylation per monosaccharide, the degree of substitution of an alkyl group represented by R¹ is 10 to 100% with 100% being defined as one substitution per monosaccharide, the degree of substitution of a carboxy-C₁₋₅ alkyl group represented by R² is 50 to 200% with 200% being defined as two substitutions per monosaccharide, and the molecular weight in terms of carboxymethyl chitin is 150,000 or smaller; or a salt thereof.

The second invention of the present application is a method of solubilizing a hydrophobic drug in which said hydrophobic drug is mixed with the chitosan derivative or a salt thereof to form a micellar aqueous composition.

"Molecular weight in terms of carboxymethyl chitin" as used herein means a molecular weight converted to those of the raw material carboxymethyl chitin before the introduction of an alkyl group of 9 to 21 carbon atoms or an aliphatic acyl group of 9 to 21 carbon atoms which is represented by R¹ in the above formula (I).

An alkyl group of 9 to 21 carbon atoms represented by R¹ in the above formula (I) may be either linear or branched, but preferably it is a linear C₉₋₂₁ alkyl group, i.e. an alkyl group represented by the formula:

-(CH₂)nCH₃, wherein n is an integer of 8 to 20,

and specifically it includes a nonyl group, a decyl group, an undecyl group, a dodecyl (lauryl) group, a tridecyl group, a tetradecyl (myristyl) group, a pentadecyl group, a hexadecyl (cetyl) group, a heptadecyl group, an octadecyl (stearyl) group, a nonadecyl group, an eicosyl group, a heneicosyl group and the like.

An aliphatic acyl group of 9 to 21 carbon atoms may be either linear or branched, or either saturated or unsaturated, but preferably it is a linear saturated C₉₋₂₁ aliphatic acyl group, i.e. an acyl group represented by the formula:

-CO(CH₂)nCH₃, wherein n is an integer of 7 to 19,

and specifically it includes a nonanoyl group, a decanoyl group, an undecanoyl group, a dodecanoyl (lauroyl) group, a tridecanoyl group, a tetradecanoyl (myristoyl) group, a pentadecanoyl group, a hexadecanoyl (palmitoyl) group, a heptadecanoyl group, an octadecanoyl (stearoyl) group, a nonadecanoyl group, an eicosanoyl group, a heneicosanoyl group and the like.

A carboxy-C₁₋₅ alkyl group represented by R² in the above formula (I) may be either linear or branched, but preferably it is a carboxy-C₁₋₅ alkyl group in which the alkyl moiety is linear, i.e. a group represented by the formula:

-(CH₂)nCOOH, wherein n is an integer of 1 to 5,

and specifically it includes a carboxymethyl group, a 2-carboxyethyl group, a 3-carboxypropyl group, a 4-carboxybutyl group, a 5-carboxypentyl group and the like.

In a chitosan derivative as used herein, the carboxyl moiety of a carboxy-C₁₋₅ alkyl group represented by R² in the above formula (I) may form a salt, preferably an alkali metal salt such as a sodium salt and a potassium salt.

A chitosan derivative as used herein may be prepared, for example, in the following manner:

First, a chitin with a molecular weight of 150,000 or smaller, preferably a molecular weight of 1,000 to 100,000, is treated with a base, preferably a hydroxide of an alkali metal such as sodium hydroxide, to prepare an alkali chitin. This is frozen at -40 to 0 °C for 5 to 24 hours and then is thawed at 0 to 40 °C, and again is frozen at -40 to 0 °C for 5 to 24 hours and then is thawed at 0 to 40 °C. Subsequently, this is reacted in the presence of a suitable solvent such as 2-propanol with a halogenated saturated fatty acid (for example, monochloroacetic acid) represented by the formula:

X-C₁₋₅ alk-COOH

wherein X is a halogen atom, preferably a chlorine atom, and alk is an alkylene group, to obtain a carboxy-C₁₋₅ alkyl chitin (for example, carboxymethyl chitin). The product is treated in the presence of a suitable solvent such as 2-propanol with a hydroxide of an alkali metal such as sodium hydroxide to obtain a carboxy-C₁₋₅ alkyl chitosan (for example, carboxymethyl chitosan). The product is then reacted in a suitable solvent such as a water-methanol mixture with an aldehyde (for example, a lauryl aldehyde) represented by the formula:

R³-CHO

wherein R³ is an alkyl group of 8 to 20 carbon atoms to obtain a chitosan derivative (for example, a laurylated carboxymethyl chitosan) in which a carboxy-C₁₋₅ alkyl group has been introduced as the hydrophilic group and an alkyl group of 9 to 21 carbon atoms has been introduced as the hydrophobic group.

Alternatively, the above carboxy-C₁₋₅ alkyl chitosan (for example, a carboxymethyl chitosan) is reacted in the presence of a suitable solvent with an acid halide (for example, lauroyl chloride) represented by the formula:

R⁴-COX

wherein R⁴ is an alkyl group of 8 to 20 carbon atoms, and X is a halogen atom preferably a chlorine atom), or with an ester (for example, ethyl laurate) represented by the formula:

R⁴-COOR⁵

wherein R⁴ is an alkyl group of 8 to 20 carbon atoms, and R⁵ is an alkyl group of 1 to 2 carbon atoms, to obtain a chitosan derivative (for example, a lauroylated carboxymethyl chitosan) in which a carboxy-C₁₋₅ alkyl group has been introduced as the hydrophilic group and an aliphatic acyl group of 9 to 21 carbon atoms has been introduced as the hydrophobic group.

Hydrophobic drugs are those drugs which cannot be formulated to an injection for pharmaceutical use or which have poor oral absorptivity due to their poor solubility in water. They include, for example, anticancer drugs such as taxol and adriamycin, antibiotics such as a macrolide, and other drugs which have poor solubility in water such as anti-inflammatory drugs, antipyretic analgesics, gastrointestinal drugs, and vitamins.

The present invention will now be explained more specifically with reference to the preferred embodiments.

The present invention provides a micellar aqueous composition comprising a hydrophobic drug and a chitosan derivative mentioned above, and a dry composition thereof.

The micellar aqueous composition as used herein means a liquid composition in which a drug has been solubilized in that the hydrophobic drug has been homogeneously dispersed in an aqueous solvent and the composition appears clear or emulsified to the naked eye. As an aqueous solvent as used herein, there may be mentioned distilled water or the like. Furthermore, it may be admixed as appropriate with a salt, a saccharide, an acid, etc., examples of which include distilled water for injection, physiological saline, a sugar solution, a transfusion solution, a buffer and the like. Furthermore, the above aqueous solvent may contain a water-soluble organic solvent, for example a small amount of ethanol etc., as long as the solvent is not toxic. The aqueous composition which was dried under a conventional drying means as appropriate is called a dry composition.

The ratio of a hydrophobic drug to the above chitosan derivative is preferably 1:0.5 to 1:5 by weight.

The means for solubilization includes, but not limited to, preferably the application of heat on swelling a chitosan derivative at a temperature of 20 to 100 °C, more preferably at 40 to 60 °C. When the drug is added to form a micelle, it is preferably subjected to sonication. The amount dissolved depends on the duration of sonication, and the duration is preferably 10 to 120 minutes, more preferably 20 to 40 minutes. For example, 2 parts by weight of a chitosan derivative is stirred under heating to swell and dissolve the polymer. Thereafter, one part by weight of the hydrophobic drug is added thereto, and the mixture is subjected to sonication to obtain a micellar aqueous composition in which the drug has been homogeneously dispersed. In preparing an injection, a sterile solution such as a sterile distilled water is used at the time of preparing a solution of an aqueous composition etc., and sterilization by filtration with a membrane-filter is conducted.

The pharmaceutical composition of the present invention may be a micellar aqueous composition or a dry composition thereof, but the dry composition is preferred since it assures the stability of hydrophobic substances during storage. As a method for preparing a dry composition, there is mentioned a method in which a solution in the form of an aqueous composition is converted to a dry composition by means of various drying methods. The drying methods include the lyophilization method, the spray drying method, the vaccum drying method, and the like, with the lyophilization method being most preferred.

The micellar aqueous composition or the dry composition thus prepared can be used as a pharmaceutical preparation such as an injection, an oral drug, a suppository, an drug for transmucosal administration, an external preparation, and the like.

### Best Mode for Carrying Out the Invention

Although the present invention will now be explained with reference to the following Preparation Examples, Working Examples, and Test Examples, it is not limited to these examples, and the manufacturing equipment, manufacturing means, the type, and the amount blended of the drug, the molecular weight, the type of the substituent, the degree of substitution, and the amount blended of the polymer may be designed as desired within the scope of the disclosure of the present invention.

### Preparation Example 1

To 2 g of crushed chitin (derived from common squid (*Todarodes pacificus*) pen) were added an aqueous solution of 55% sodium hydroxide (8 ml) and an aqueous solution of 8% sodium dodecylsulfate (200 µl), and the mixture was stirred at 4 °C for 2 hours to prepare an alkali chitin. This was frozen at -20 °C for 8 hours and then thawed at room temperature, which was frozen again at -20 °C for 8 hours and then thawed at room temperature. To the product was added 2-propanol (30 ml), and monochloroacetic acid was added thereto while stirring till the solution becomes neutral to obtain a crude carboxymethyl chitin. The chitin obtained was then subjected to washing with methanol, dialysis, and filtration to obtain a purified carboxymethyl chitin, to which were added an aqueous solution of 45% sodium hydroxide (40 ml) and 2-propanol (40 ml) and the resulting mixture was refluxed at 110 °C for 1 hour. After the 2-propanol was removed and the aqueous layer was neutralized with hydrochloric acid, a crude carboxymethyl chitosan was allowed to deposit with methanol. This was dialyzed to obtain a pure carboxymethyl chitosan, to which were added water (40 ml) and methanol (40 ml). Lauryl aldehyde (dodecanal) (4 g) was added thereto, and was stirred for 30 minutes, and then sodium borohydride (1.3 g) was added and stirred at room temperature for 8 hours. Acetone was added to this and the precipitate thus obtained was washed with methanol and hexane, dialyzed and then lyophilized to obtain laurylated carboxymethyl chitosan (0.75 g).

The physical characteristics of the laurylated carboxymethyl chitosan obtained is as shown in Table 1 below:

**Table 1**

| Physical characteristics of laurylated carboxymethyl chitosan | | | |
|---|---|---|---|
| Molecular weight * | Degree of laurylation (%) | Degree of carboxymethylation (%) | Degree of deacetylation (%) |
| 50000 | 90 | 140 | 100 |

| | | | |
|---|---|---|---|
| * Molecular weight: the molecular weight of carboxymethyl chitin | | | |

The physical characteristics (molecular weight, the degree of deacetylation, the degree of carboxymethylation, and the degree of laurylation) of chitins and their derivatives were measured in the following methods, respectively.

The molecular weight of carboxymethyl chitin was determined by the method of Inoue et al. (Inoue Y., Kaneko M. and Tokura S., Rep. Progr. Polym. Phys. Jap., 25, 759 (1982)). The degree of deacetylation, the degree of carboxymethylation, and the degree of laurylation for chitins and their derivatives were determined using an elementary analysis instrument (2400 CHN elementary analysis instrument, manufactured by Perkin-Elmer), and the degree of substitution was calculated from the elementary compositions.

### Preparation Examples 2 and 3

A similar procedure as in Preparation Example 1 was followed except that reaction times, reaction temperatures and the like were controlled to prepare laurylated carboxymethyl chitosans with different degrees of laurylation, carboxymethylation, and deacetylation.

The physical characteristics of the laurylated carboxymethyl chitosan obtained is as shown in Table 2.

### Preparation Examples 4 to 6

The carboxymethyl chitin (4 g) obtained as an intermediate in Preparation Example 1 was stirred in concentrated hydrochloric acid (100 ml) at room temperature for 1 hour. This was neutralized and the deposited low molecular weight carboxymethyl chitin was obtained. Using this as a raw material in a method similar to that in Preparation Example 1, laurylated carboxymethyl chitosan (0.8 g) was obtained.

The physical characteristics of the laurylated carboxymethyl chitosan obtained is as shown in Table 2.

**Table 2**

| Physical characteristics of laurylated carboxymethyl chitosan | | | | |
|---|---|---|---|---|
| | Molecular weight * | Degree of laurylation (%) | Degree of carboxymethylation (%) | Degree of deacetylation (%) |
| Prep. Ex. 2 | 50000 | 65 | 140 | 100 |
| Prep. Ex. 3 | 50000 | 19 | 200 | 90 |
| Prep. Ex. 4 | 2600 | 25 | 140 | 100 |
| Prep. Ex. 5 | 2300 | 25 | 140 | 100 |
| Prep. Ex. 6 | 2000 | 25 | 140 | 100 |

| | | | | |
|---|---|---|---|---|
| * Molecular weight: the molecular weight of carboxymethyl chitin | | | | |

### Working Example 1

About 10 mg of adriamycin hydrochloride was weighed out, 1 ml of water was added thereto, and the mixture was stirred and dissolved. Then about 5 mg of sodium bicarbonate was added while stirring to obtain a suspension of free adriamycin.

Separately, about 10 mg of laurylated carboxymethyl chitosan obtained in Preparation Example 1 (the molecular weight of carboxymethyl chitin: 50000, sodium salt, R¹: lauryl, R²: carboxymethyl, the degree of laurylation: 90%, the degree of carboxymethylation: 140%, the degree of deacetylation: 100%) was weighed out, 9 ml of 0.15 M phosphate buffer (pH 7.4) was added thereto and the chitosan derivative was allowed to swell by stirring at 50 °C for 4 to 5 hours.

To this was added a total amount of the previously prepared suspension of adriamycin, and the mixture was subjected to sonication using a probe-type sonifier (manufactured by Branson, sonifier type 250, 20 kHz, 20 to 30 W) for 30 minutes. It was then filtered with a 5 µm membrane filter in order to remove the precipitate. To remove adriamycin dissolved in the filtrate, it was dialyzed overnight against 0.15 M phosphate buffer (pH 7.4) (molecular weight cutoff (MWCO) 3500), and was further centrifuged to remove the precipitate etc. A red emulsified micellar composition of laurylated carboxymethyl chitosan in which adriamycin has been included was obtained.

### Comparative Example 1

About 50 mg of adriamycin hydrochloride was weighed out, 5 ml of water was added thereto, and the mixture was dissolved. To this was added about 25 mg of sodium bicarbonate while stirring. To remove the red precipitate that deposited, it was filtered with a 5 µm membrane filter. A pale red saturated solution of adriamycin was obtained as the filtrate.

### Comparative Example 2

About 10 mg of laurylated carboxymethyl chitosan obtained in Preparation Example 1 (the molecular weight of carboxymethyl chitin: 50000, sodium salt, R¹: lauryl, R²: carboxymethyl, the degree of laurylation: 90%, the degree of carboxymethylation: 140%, the degree of deacetylation: 100%) was weighed out, 10 ml of 0.15 M phosphate buffer (pH 7.4) was added thereto, and the chitosan derivative was allowed to swell by stirring at 50 °C for 4 to 5 hours. The mixture was subjected to sonication using a probe-type sonifier (manufactured by Branson, sonifier type 250, 20 kHz, 20 to 30 W) for 30 minutes. It was then filtered with a 5 µm membrane filter in order to remove the precipitate. An emulsified micellar composition of laurylated carboxymethyl chitosan was obtained as the filtrate.

The solutions obtained in Working Example 1, Comparative Example 1, and Comparative Example 2 were concentrated (3000 rpm, 20 minutes) in centrifuge tubes (Centricon, manufactured by Grace Japan, MWCO 10000) equipped with an ultrafiltration membrane. The concentrated solution that remained in the tube without passing through the membrane was obtained in the upper layer and the filtrate was obtained in the bottom layer. Based on the colors of the upper layer and the bottom layer of the Centricon tube, inclusion of adriamycin was evaluated.

As a result, in the case of Comparative Example 1, a pale red color resulting from adriamycin was observed in both the upper layer and the bottom layer. This indicated that dissolved adriamycin passes through the ultrafiltration membrane (MWCO 10000).

In Comparative Example 2, the upper layer showed a milky white color resulting from the micelle, and the bottom layer was clear and colorless. This indicated that the micelle does not pass through the ultrafiltration membrane (MWCO 10000).

Furthermore, in Working Example 1 the upper layer was a red emulsion, and the bottom layer was clear and colorless.

If adriamycin is dissolved in the molecular form, it would pass through the ultrafiltration membrane. Therefore, in the micelle solution of Working Example 1 it was shown that adriamycin is not dissolved in the molecular form and that adriamycin has been included in the micelle. Furthermore, since the red color in the upper layer is evidently deeper than that in the upper layer of the adriamycin solution of Comparative Example 1, it was confirmed that adriamycin was solubilized more than the saturation solubility by being included in the laurylated carboxymethyl chitosan.

### Working Example 2

To 10 mg of laurylated carboxymethyl chitosan obtained in Preparation Example 1 (the molecular weight of carboxymethyl chitin: 50000, sodium salt, the degree of laurylation: 90%, the degree of carboxymethylation: 140%, the degree of deacetylation: 100%) was added 9 ml of 0.15 M phosphate buffer (pH 7.4), and the mixture was stirred at 50 °C for 2 to 3 hous to allow the chitosan derivative to swell. Then, 1 ml of solution of taxol in ethanol (5 mg/ml) was added. The mixture was solubilized by sonication using a probe-type sonifier (manufactured by Branson, sonifier type 250, 20 kHz, 20 to 30 W) for 30 minutes to obtain a slightly milky white solution. It was then dialyzed (MWCO 3500) overnight against a phosphate buffer (pH 7.4) and was filtered with a 5 µm membrane filter to obtain a micellar aqueous composition.

### Working Example 3

To 50 mg of laurylated carboxymethyl chitosan obtained in Preparation Example 1 (the molecular weight of carboxymethyl chitin: 50000, sodium salt, the degree of laurylation: 90%, the degree of carboxymethylation: 140%, the degree of deacetylation: 100%) was added 9 ml of 0.15 M phosphate buffer (pH 7.4), and the mixture was stirred at 50 °C for 2 to 3 hous to allow the chitosan derivative to swell. Then, 1 ml of solution of taxol in ethanol (30 mg/ml) was added. Subsequently, a similar procedure as in Working Example 2 was followed to obtain a micellar aqueous composition.

### Working Example 4

Using laurylated carboxymethyl chitosan obtained in Preparation Example 2 (the molecular weight of carboxymethyl chitin: 50000, sodium salt, the degree of laurylation: 65%, the degree of carboxymethylation: 140%, the degree of deacetylation: 100%), a similar procedure as in Working Example 3 were followed to obtain a micellar aqueous composition.

### Working Example 5

Using laurylated carboxymethyl chitosan obtained in Preparation Example 3 (the molecular weight of carboxymethyl chitin: 50000, sodium salt, the degree of laurylation: 19%, the degree of carboxymethylation: 200%, the degree of deacetylation: 90%), a similar procedure as in Working Example 3 was followed to obtain a micellar aqueous composition.

### Working Example 6

Using low molecular weight laurylated carboxymethyl chitosan obtained in Preparation Example 4 (the molecular weight of carboxymethyl chitin: 2600, sodium salt, the degree of laurylation: 25%, the degree of carboxymethylation: 140%, the degree of deacetylation: 100%), a similar procedure as in Working Example 3 was followed to obtain a micellar aqueous composition.

### Working Example 7

Using low molecular weight laurylated carboxymethyl chitosan obtained in Preparation Example 5 (the molecular weight of carboxymethyl chitin: 2300, sodium salt, the degree of laurylation: 25%, the degree of carboxymethylation: 140%, the degree of deacetylation: 100%), a similar procedure as in Working Example 3 was followed to obtain a micellar aqueous composition.

### Working Example 8

Using low molecular weight laurylated carboxymethyl chitosan obtained in Preparation Example 6 (the molecular weight of carboxymethyl chitin: 2000, sodium salt, the degree of laurylation: 25%, the degree of carboxymethylation: 140%, the degree of deacetylation: 100%), a similar procedure as in Working Example 3 was followed to obtain a micellar aqueous composition.

### Comparative Example 3

Using carboxymethyl chitin (the molecular weight of carboxymethyl chitin: 50000, sodium salt, R¹ = H, R²: carboxymethyl), a similar procedure as in Working Example 2 was followed.

### Test Example

Concerning quantitation, 500 µl of each pharmaceutical preparation was lyophilized, to which was added 10 ml of an internal standard (an ethanol solution of dibutyl phthalate 20 µg/ml). The mixture was subjected to sonication for 5 minutes and centrifuged (3000 rpm, 5 minutes) to obtain a supernatant as the sample solution. After diluting with ethanol, as desired, the content of taxol for 10 µl of the sample solution was determined by high performance liquid chromatography (HPLC).

### HPLC conditions:

Column: ODS for liquid chromatography with a diameter of 5 µm was packed into a stainless steel column of 4.6 mm in diameter x 15 cm
Column temperature: 50 °C
Packing material: ODS80Tm (Tosoh)
Mobile phase: methanol : water = 65 : 35
Detection wavelength: 227 nm
Flow rate: 1.0 ml/min
Amount injected: 10 µl

For the pharmaceutical preparations of Working Examples 2 - 8 and Comparative Example 3, the preparation was diluted appropriately in a phosphate buffer (pH 7.4) to determine the particle size by the dynamic light scattering method.

### Particle size measurement condition:

Instrument: DLS7000 (Otuka Electronics)
Diluting solution: phosphate buffer (pH 7.4)
Frequency of integration: 200 times
Sampling time: 8 µsec
Method of analysis: histogram method (Marquadt method)
Expression of distribution: weight distribution

The chitosan derivatives used are summarized in Table 3. The results are shown in Table 4.

**Table 3**

| Chitosan derivatives | | | | |
|---|---|---|---|---|
| | Molecular weight * | Degree of laurylation (%) | Degree of carboxymethylation (%) | Degree of deacetylation (%) |
| Work. Ex. 2 | 50000 | 90 | 140 | 100 |
| Work. Ex. 3 | 50000 | 90 | 140 | 100 |
| Work. Ex. 4 | 50000 | 65 | 140 | 100 |
| Work. Ex. 5 | 50000 | 19 | 200 | 90 |
| Work. Ex. 6 | 2600 | 25 | 140 | 100 |
| Work. Ex. 7 | 2300 | 25 | 140 | 100 |
| Work. Ex. 8 | 2000 | 25 | 140 | 100 |
| Comp. Ex. 3 | 50000 | 0 | 140 | 100 |

| | | | | |
|---|---|---|---|---|
| * Molecular weight: the molecular weight of carboxymethyl chitin | | | | |

**Table 4**

| Results of Working Examples 2 - 8 | | | | | |
|---|---|---|---|---|---|
| | Amount added | | Filtration condition | Results | |
| | Chitin derivative (mg) | Taxol (mg) | Membrane filtration (µm) | Taxol conc. (µg/ml) | Mean particle size (nm) |
| Work. Ex. 2 | 10 | 5 | 5 | 250 | 32 |
| Work. Ex. 3 | 50 | 30 | 5 | 2500 | 91 |
| Work. Ex. 4 | 50 | 30 | 0.8 | 700 | 51 |
| Work. Ex. 5 | 50 | 30 | 0.8 | 1400 | 70 |
| Work. Ex. 6 | 50 | 30 | 5 | 1700 | 42 |
| Work. Ex. 7 | 50 | 30 | 0.8 | 1400 | 63 |
| Work. Ex. 8 | 50 | 30 | 5 | 1600 | 60 |
| Comp. Ex. 3 | 50 | 5 | 5 | lower than detection limit | not detected |

Although the saturation solubility of taxol is 0.32 µg/ml at pH 7.4, the amount solubilized remarkably increased in Working Examples 2 - 8. The particle size distributions of the micelles have shown an almost single peak in terms of weight distribution for all Working Examples with a mean particle size of about 10 nm, and it was solubilized by forming a micellar aqueous composition. Each micellar aqueous composition has turned a clear or a slightly milky white solution.

### Industrial Applicability

Hydrophobic drugs were solubilized using chitosan derivatives in which carboxy-C₁₋₅ alkyl groups have been introduced as the hydrophilic groups and alkyl groups of 9 to 21 carbon atoms or aliphatic acyl groups of 9 to 21 carbon atoms have been introduced as the hydrophobic groups. For taxol, in particular, solubilization exceeded the saturation solubility by a factor of several thousand. The present invention is not limited to taxol but can be applied to other hydrophobic drugs, and thereby enables the formulation of drugs as injections that could not be used for injection due to its insolubility in water, and also the effect of promoting absorptivity of drugs that were poorly absorbed orally due to its poor solubility can be expected.

## Claims

1. A pharmaceutical composition comprising
(1) a hydrophobic drug, and
(2) a chitosan derivative represented by formula (I): 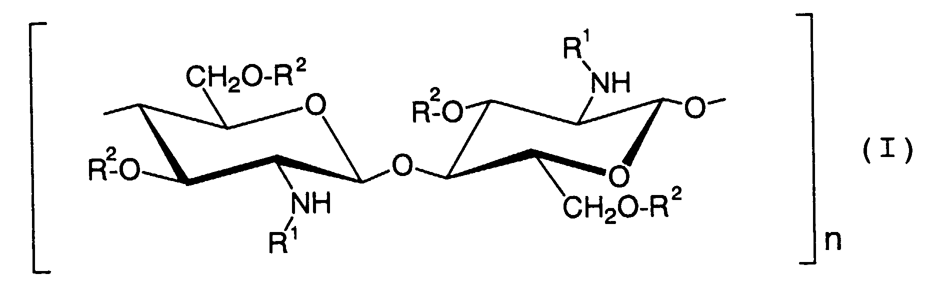
wherein R¹ represents a hydrogen atom, an acetyl group, an alkyl group of 9 to 21 carbon atoms, or an aliphatic acyl group of 9 to 21 carbon atoms, and R² represents a hydrogen atom or a carboxy-C₁₋₅ alkyl group; and
the degree of deacetylation of an N-acetyl group represented by R¹ is 70 to 100% with 100% being defined as one deacetylation per monosaccharide, the degree of substitution of an alkyl group represented by R¹ is 10 to 100% with 100% being defined as one substitution per monosaccharide, the degree of substitution of a carboxy-C₁₋₅ alkyl group represented by R² is 50 to 200% with 200% being defined as two substitutions per monosaccharide, and the molecular weight in terms of carboxymethyl chitin is 150,000 or smaller; or a salt thereof.

2. A pharmaceutical composition according to claim 1 in which said hydrophobic drug is adriamycin or taxol.

3. A pharmaceutical composition according to claim 1 which is a micellar aqueous composition.

4. A pharmaceutical composition according to claim 1 which is a dry composition and which forms a micelle when dissolved in an aqueous solvent.

5. A method of solubilizing a hydrophobic drug wherein said method comprises mixing said hydrophobic drug with:
a chitosan derivative represented by formula (I): 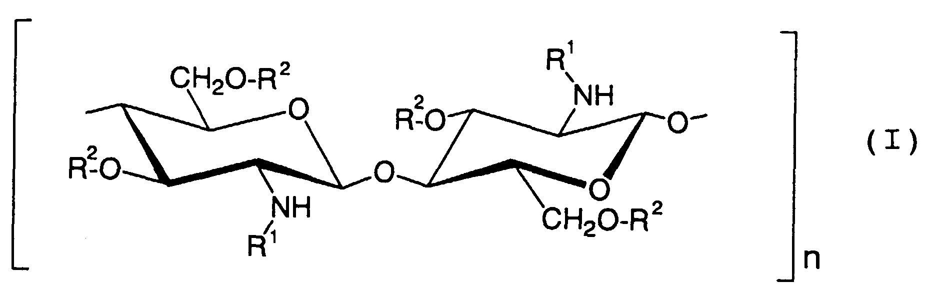
wherein R¹ represents a hydrogen atom, an acetyl group, an alkyl group of 9 to 21 carbon atoms, or an aliphatic acyl group of 9 to 21 carbon atoms, and R² represents a hydrogen atom or a carboxy-C₁₋₅ alkyl group; and
the degree of deacetylation of an N-acetyl group represented by R¹ is 70 to 100% with 100% being defined as one deacetylation per monosaccharide, the degree of substitution of an alkyl group represented by R¹ is 10 to 100% with 100% being defined as one substitution per monosaccharide, the degree of substitution of a carboxy-C₁₋₅ alkyl group represented by R² is 50 to 200% with 200% being defined as two substitutions per monosaccharide, and the molecular weight in terms of carboxymethyl chitin is 150,000 or smaller; or a salt thereof to form a micellar aqueous composition. 6. The method of solubilization according to claim 5 in which said hydrophobic drug is adriamycin or taxol.
